# DEMANDE DE BREVET EUROPEEN

(11) **EP 1 739 085 A1**
(43) Date de publication de la demande: **03.01.2007**
(21) Numéro de dépôt: 06116073.5
(22) Date de dépôt: 26.06.2006
(51) Int. Cl.: C07D 401/04, C07D 333/46, C07D 307/34, C07D 213/06, C07C 211/51

(54) **Compositions cosmétique contenant une para-phénylènediamines doubles reliées par un groupe aromatique et utilisation en coloration**

(30) Priorité: 29.06.2005 FR 0551806
(71) Demandeur: L'ORÉAL, 75008 Paris (FR)
(72) Inventeur: Sabelle, Stéphane, 75005 Paris (FR); Metais, Eric, 95320, St Leu la Forêt (FR)
(74) Mandataire: Fevrier, Murielle Françoise E.

(57) **Abrégé**

La présente demande concerne une nouvelle famille de para-phénylènediamines doubles reliées par un bras de liaison comportant un groupement aromatique et leur utilisation pour la coloration des fibres kératiniques.

Ces nouvelles para-phénylènediamines doubles de formule (I) sont utiles en tant que base d'oxydation pour la coloration des fibres kératiniques.

## Description

La présente demande concerne une nouvelle famille de para-phénylènediamines doubles reliées par un bras de liaison comportant un groupement aromatique et leur utilisation pour la coloration des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques et pyridiniques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

Le but de la présente invention est de fournir de nouvelles bases d'oxydation capables de colorer les fibres kératiniques avec des nuances variées, puissantes, esthétiques et peu sélectives ainsi que des colorations résistantes aux diverses agressions que peuvent subir les fibres telles que la lumière, la sueur et les shampoings.

Ce but est atteint avec la présente invention qui a pour objet une composition cosmétique de coloration des fibres kératiniques comprenant dans un milieu cosmétique approprié pour la coloration au moins une base d'oxydation para-phénylènediamine double de formule (I) suivante ainsi que les sels d'addition correspondants : 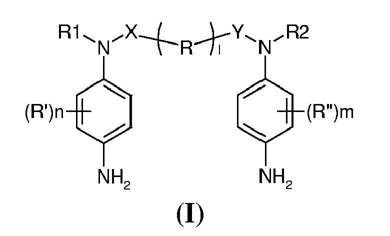 dans laquelle :
- R représente un cycle aromatique à 5 ou 6 chaînons contenant éventuellement un ou plusieurs atomes d'azote, de soufre et d'oxygène, ce cycle pouvant être substitué, notamment par un ou plusieurs radicaux choisis parmi les radicaux alkyles en C₁-C₄ ou hydroxyles, ou hydroxyalkyles,
- R₁ et R₂ représentent, indépendamment l'un de l'autre,
   - un atome d'hydrogène,
   - un radical alkyle en C₁-C₆linéaire ou ramifié, pouvant être substitué par un ou plusieurs radicaux choisis parmi les radicaux hydroxy, alkoxy en C₁-C₄, amino, mono-alkylamino en C₁-C₄, dialkylamino en C₁-C₄,
- X et Y représentent, indépendamment l'un de l'autre, un radical alkylène en C₁-C₁₀ linéaire ou ramifié,
- R' et R" représentent, indépendamment l'un de l'autre,
   - un radical alkyle en C₁-C₆,
   - un radical alcoxy en C₁-C₆,
   - un radical hydroxy-alcoxy en C₁-C₆,
   - un radical alcoxy (C₁-C₆)alkyle en C₁-C₆,
   - un radical mono ou poly-hydroxy alkyle en C₁-C₆,
- n et m représentent, indépendamment l'un de l'autre, un nombre entier variant de 0 à 4, et
- 1 représente un nombre entier égal à 1 ou 2.

L'invention a aussi pour objet un procédé de coloration mettant en oeuvre cette composition, l'utilisation de la composition selon la présente invention pour la coloration des fibres kératiniques, en particulier les fibres kératiniques humaines telles que les cheveux et un dispositif à plusieurs compartiments ou "kit" de coloration.

La présente invention a pour objet les nouvelles para-phénylènediamines de formule (I) telle que définie précédemment à l'exception des para-phénylènediamines de formule :

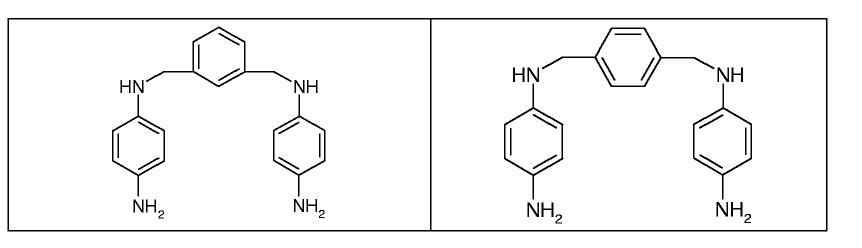

ainsi que les composés nitrés correspondants de formule (II) suivantes : 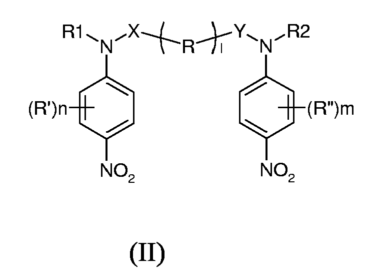 dans laquelle R₁, R₂, X, Y, R, R', R", n, m et 1 sont tels que définis précédemment à l'exception des composés nitrés suivants :

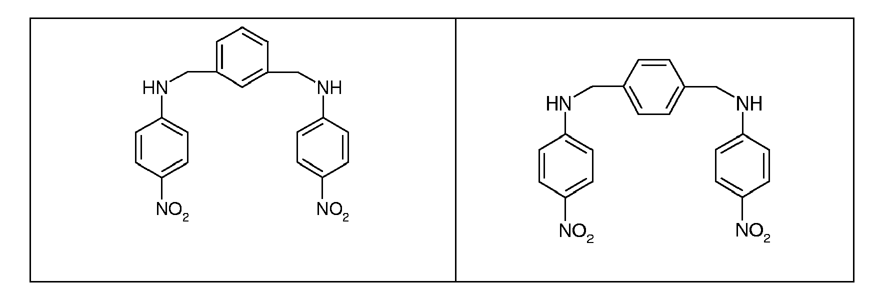

Les composés nitrés de formule (II) permettent d'obtenir par réduction les para-phénylènediamines de formule (I).

La composition de la présente invention comprenant les para-phénylènediamines de formule (I) permet en particulier d'obtenir une coloration de fibres kératiniques très puissante, peu sélective et tenace, en particulier à la lumière.

A titre d'exemple, la composition de coloration de l'invention peut contenir à titre de para-phénylènediamines de formule (I) les para-phénylènediamines suivantes :

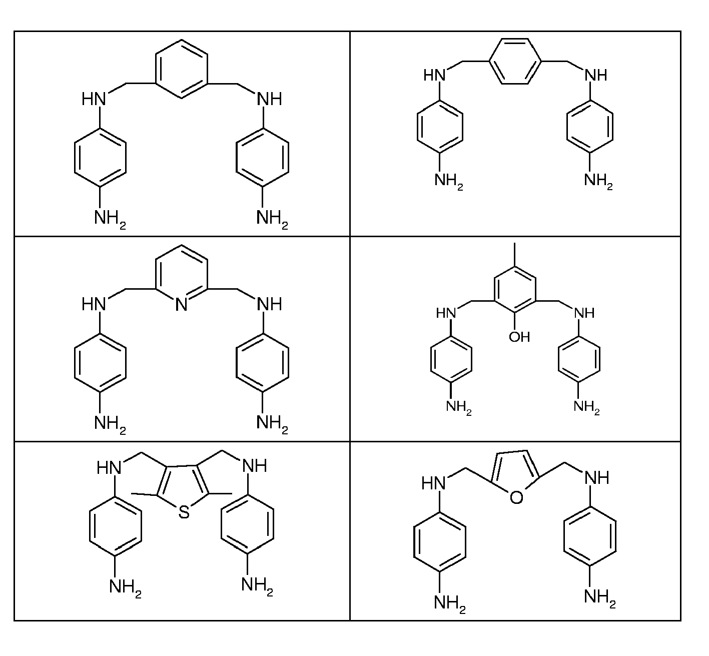

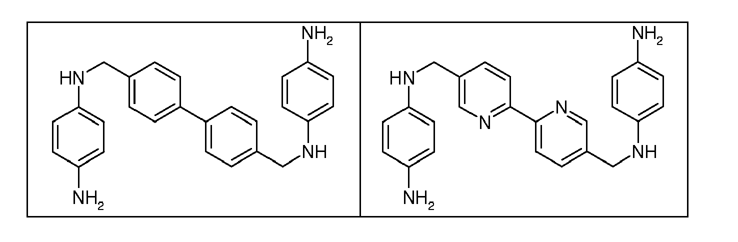

Selon un mode de réalisation particulier, la composition de l'invention comprend les para-phénylènediamines de formule (I) telles que R est un radical phénylène pouvant être substitué. De préférence, R₁ et R₂ représentent indépendamment un atome d'hydrogène ou un groupe alkyle en C₁-C₄ pouvant être substitué, X et Y représentent indépendamment un radical alkylène en C₁-C₃. n et m sont de préférence égal à 0 ou 1 et 1 est de préférence égal à 1.

D'une manière générale, les sels d'addition utilisables dans la composition de l'invention sont notamment choisis parmi les sels d'addition avec un acide, tels que l'acide chlorhydrique, l'acide bromhydrique, l'acide sulfurique, l'acide citrique, l'acide succinique, l'acide tartrique, l'acide lactique, l'acide para-toluènesulfonique, l'acide benzènesulfonique, l'acide phosphorique et l'acide acétique.

Ils peuvent aussi être sous forme de solvates par exemple un hydrate ou un solvate d'alcool linéaire ou ramifié tel que l'éthanol ou l'isopropanol.

A titre de para-phénylènediamines préférées dans la composition de coloration de l'invention, on peut citer

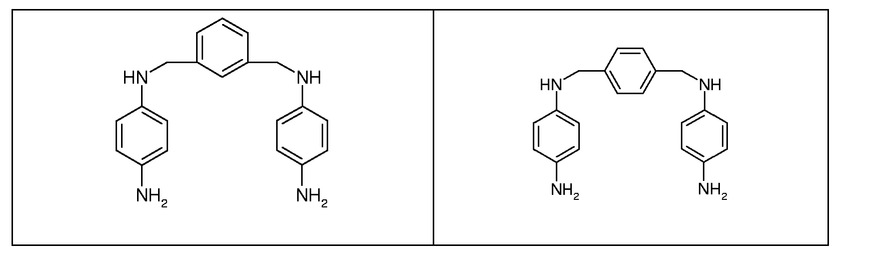

La quantité en paraphénylènediamine de formule (I) dans la composition de coloration est en général comprise entre 0,0001 % et 20 % en poids par rapport au poids total de la composition, de préférence entre 0,01 % et 10 %.

La composition selon l'invention contient de préférence au moins un coupleur d'oxydation.

Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la quantité du ou des coupleurs d'oxydation est comprise entre 0,0001 et 20%, de préférence entre 0,005 à 6% en poids par rapport au poids total de la composition.

La composition selon l'invention peut également contenir au moins une base d'oxydation additionnelle différente des bases d'oxydation de formule (I).

Les bases d'oxydation peuvent notamment être choisies parmi les para-phénylènediamines, les bis-phénylalkylènediamines, les para--aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4 aminophenyl pyrrolidine, le 2 thiényl para-phénylène diamine, le 2-β hydroxyéthylamino 5-amino toluène, la 3-hydroxy 1-(4' aminophenyl)pyrrolidine, la 6-(4-Amino-phenylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, la 6-(4-Amino-phenylamino)-hexan-1-ol et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4'-aminophényl) tétraméthylènediamine, la N,N'-bis-(β-hydroxyéthyl) N,N'-bis-(4'-aminophényl) tétraméthylènediamine, la N,N'-bis-(4'-amino-3'-méthylphényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6-dichlorophénol, le 4-amino-6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, le 5-[(2-hydroxyéthyl)amino]-2-méthylphenol et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1026978 et GB 1153196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2733749 et DE 19543988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation additionnelle est comprise entre 0,0001 et 20%, de préférence entre 0,005 à 6% en poids par rapport au poids total de la composition.

La composition tinctoriale conforme à l'invention peut en outre contenir un ou plusieurs colorants directs pouvant notamment être choisi parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention, on peut citer les colorants directs azoïques cationiques décrits dans les demandes de brevets WO 95/15144, WO-95/01772 et EP-714954.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Le milieu cosmétique approprié pour la coloration des fibres kératiniques est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40% en poids environ par rapport au poids total de la composition de coloration, et encore plus préférentiellement entre 5 et 30% en poids environ.

Avantageusement, la composition de coloration comprend au moins un adjuvant cosmétique choisi parmi les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20% en poids par rapport au poids de la composition.

Le pH de la composition conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en coloration des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 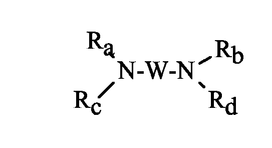 dans laquelle W est un reste propylène éventuellement substitué par un groupement hydroxyle ou un radical alkyle en C₁-C₄; Rₐ, R_{b}, R_{c} et R_{d}, identiques ou différents, représentent un atome d'hydrogène, un radical alkyle en C₁-C₄ ou hydroxyalkyle en C₁-C₄.

Bien entendu, l'homme de l'art veillera à choisir le ou les adjuvants, précurseurs de colorants d'oxydations additionnels, coupleurs d'oxydation, colorants directs de manière telle que les propriétés avantageuses attachées intrinsèquement à la composition de coloration d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une coloration des fibres kératiniques, et notamment des cheveux humains.

Un autre objet de la présente demande concerne un procédé de coloration des fibres kératiniques dans lequel on applique sur les fibres kératiniques la composition de l'invention telle que définie précédemment, et qu'on révèle la couleur à l'aide d'un agent oxydant. La couleur peut être révélée à pH acide, neutre ou alcalin. L'agent oxydant peut être ajouté à la composition de l'invention juste au moment de l'emploi. Il peut être mis en oeuvre à partir d'une composition oxydante le contenant, appliquée simultanément ou séquentiellement à la composition de l'invention.

A titre d'agents oxydants, on peut citer le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, le peroxyde d'hydrogène étant particulièrement préféré.

Selon un mode de réalisation particulier, la composition selon la présente invention est mélangée, de préférence au moment de l'emploi, à une composition contenant, dans un milieu approprié pour la coloration, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut renfermer divers adjuvants utilisés classiquement dans les compositions pour la coloration des cheveux et tels que défmis précédemment.

Le pH de la composition oxydante renfermant l'agent oxydant est tel qu'après mélange avec la composition de coloration, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés pour la coloration des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une coloration des fibres kératiniques, notamment humaines et en particulier les cheveux humains.

La présente demande concerne également l'utilisation de la composition selon l'invention comprenant, dans un milieu approprié pour la coloration, au moins une paraphénylènediamine de formule (I) pour la coloration des fibres kératiniques, de préférence les fibres kératiniques humaines telles que les cheveux.

L'invention a aussi pour objet un dispositif à plusieurs compartiments ou "kit" de coloration dans lequel un premier compartiment renferme une composition comprenant au moins une base d'oxydation de formule (I) telle que définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2586913 au nom de la demanderesse.

Les para-phénylènediamines de formule (I) selon la présente demande peuvent être préparées suivant une méthode de synthèses classique. On pourra par exemple se reporter à la demande de brevet DE10144226A.

A titre d'illustration, les para-phénylènediamines de formule (I) peuvent être synthétisées selon le schéma réactionnel suivant :

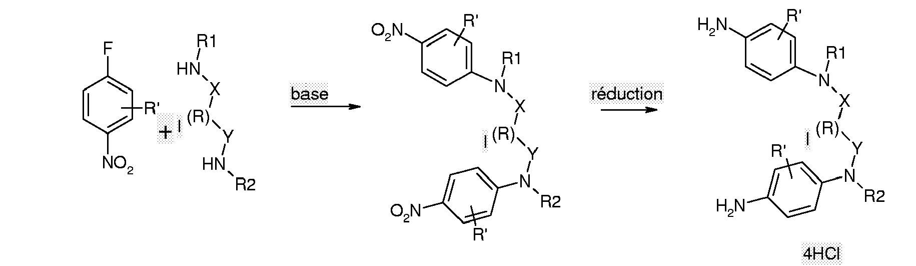

La première étape de la synthèse est une substitution nucléophile d'une diamine sur un dérivé de para-fluoro-nitrobenzène, étape inspirée des publications Synthesis 1990 (12), 1147-1148 et Synth. Commun. 1990, 20 (22), 3537-3545. La deuxième étape est une étape de réduction classique, pouvant être par exemple une réaction d'hydrogénation par catalyse hétérogène en présence de Pd/C, Pd(II)/C, Ni de Raney ou encore une réaction de réduction par un métal, par exemple par du zinc, fer, étain... (Advanced Organic Chemistry, 4th edition, 1992, J. MARCH, WILEY Interscience ; Reduction in Organic Chemistry, M. Hudlicky, 1983, Ellis Honwood series Chemical Science).

### EXEMPLES DE SYNTHESE

### EXEMPLE 1 : Synthèse du tétrachlorhydrate de N-(3-{[(4-aminophényl)amino]méthyl}benzyl)benzène-1,4-diamine (2)

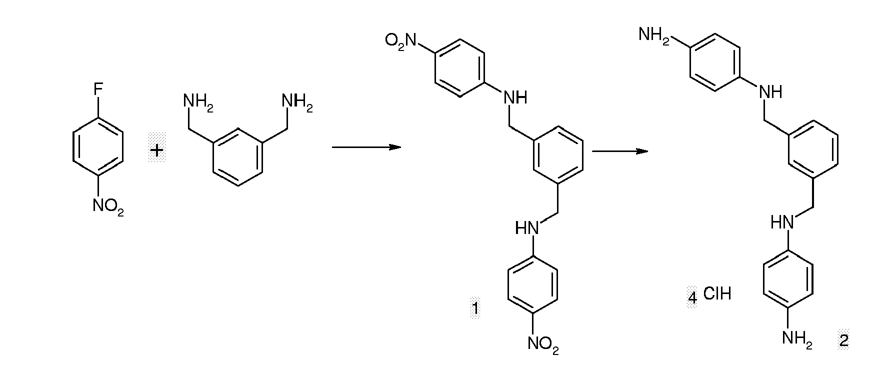

### Etape 1 : synthèse de 4-nitro-N-(3-{[(4-nitrophépyl)aminol] méthyl}benzyl)aniline (1)

1,73 g de para-fluoronitrobenzene (2éq.) sont dissous dans 5 ml de DMSO. 1,2 équivalents de m-xylène diamine et 4 équivalents de triéthylamine sont ajoutés à la solution. Le milieu réactionnel est porté à 60°C pendant 20 heures. Le mélange est ensuite versé sur de la glace pilée, un précipité se forme. Ce dernier est filtré, lavé à l'eau, puis séché.

### Etape 2 : synthèse du tétrachlorhydrate de N-(3-{[(4-aminophényl)amino] méthyl}benzyl)benzène-1,4-diamine (2)

Le mélange de zinc en poudre (30 g), le chlorure d'ammonium (3 g), l'eau (5 ml) et l'éthanol absolue sont portés au reflux avec le bain d'huile. Le 4-nitro-N-(3-{[(4-nitrophényl)amino]méthyl}benzyl)aniline (3 g) est dissous dans 50 ml de NMP et introduit goutte à goutte jusqu'à décoloration du milieu réactionnel tout en maintenant le reflux.

On filtre à chaud sur célite et on récupère le filtrat dans un mélange éthanol et acide chlorhydrique concentré. Après ajout d'isopropanol, on concentre sous vide jusqu'à précipitation d'un solide correspondant au tétrachlorhydrate du N-(3-{[(4-aminophényl)amino]méthyl}benzyl)benzène-1,4-diamine (2).

Les spectres RMN du proton et de masse sont conformes à la structure attendue du produit.

### EXEMPLE 2: Synthèse du tétrachlorhydrate de la N-(4-{[(4-aminophényl)amino]méthyl}benzyl)benzène-1,4-diamine (4)

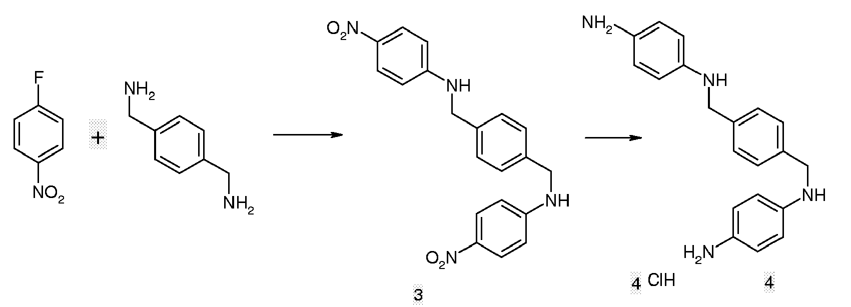

### Etape 1 : synthèse de la 4-nitro-N-(4-{[(4-nitrophényl)amino]méthyl}benzyl) aniline (3)

4 g de para-fluoronitrobenzene (2éq.) sont dissous dans 5 ml de DMSO. 1,2 équivalent de α,α diamino-p-xylène et 4 équivalents de triéthylamine sont ajoutés à la solution. Le milieu réactionnel est porté à 60°C pendant 24 heures. Le mélange est ensuite versé sur de la glace pilée, un précipité se forme. Ce dernier est filtré, lavé à l'eau, puis séché.

### Etape 2 : synthèse du tétrachlorhydrate de la N-(4-{[(4-aminophényl)amino]méthyl}benzyl)benzène-1,4-diamine (4)

Le mélange de zinc en poudre (22,2 g), le chlorure d'ammonium (2,5 g), l'eau (4 ml) et l'éthanol absolue (20 ml) sont portés au reflux avec le bain d'huile. Le 4-nitro-N-(4-{[(4-nitrophényl) amino]méthyl}benzyl)aniline (3,4 g) est dissous dans 20 ml de NMP et introduit goutte à goutte jusqu'à décoloration du milieu réactionnel tout en maintenant le reflux.

On filtre à chaud sur célite et on récupère le filtrat dans une mélange éthanol et acide chlorhydrique concentre. Après ajout d'isopropanol, on concentre sous vide pour obtenir un solide correspondant au tétrachlorhydrate de la N-(4-{[(4-aminophényl)amino]méthyl}benzyl)benzène-1,4-diamine (4).

Les spectres RMN du proton et de masse sont conformes à la structure attendue du produit.

### EXEMPLES DE TEINTURE

### Exemples 1 à 14 : Composition tinctoriale à partir du tétrachlorhydrate de N-(4-{[(4-aminophényl)amino]méthyl}benzyl)benzène-1,4-diamine (4)

### Exemples 1 à 7 : Teinture en milieu acide

Les compositions tinctoriales suivantes sont préparées :

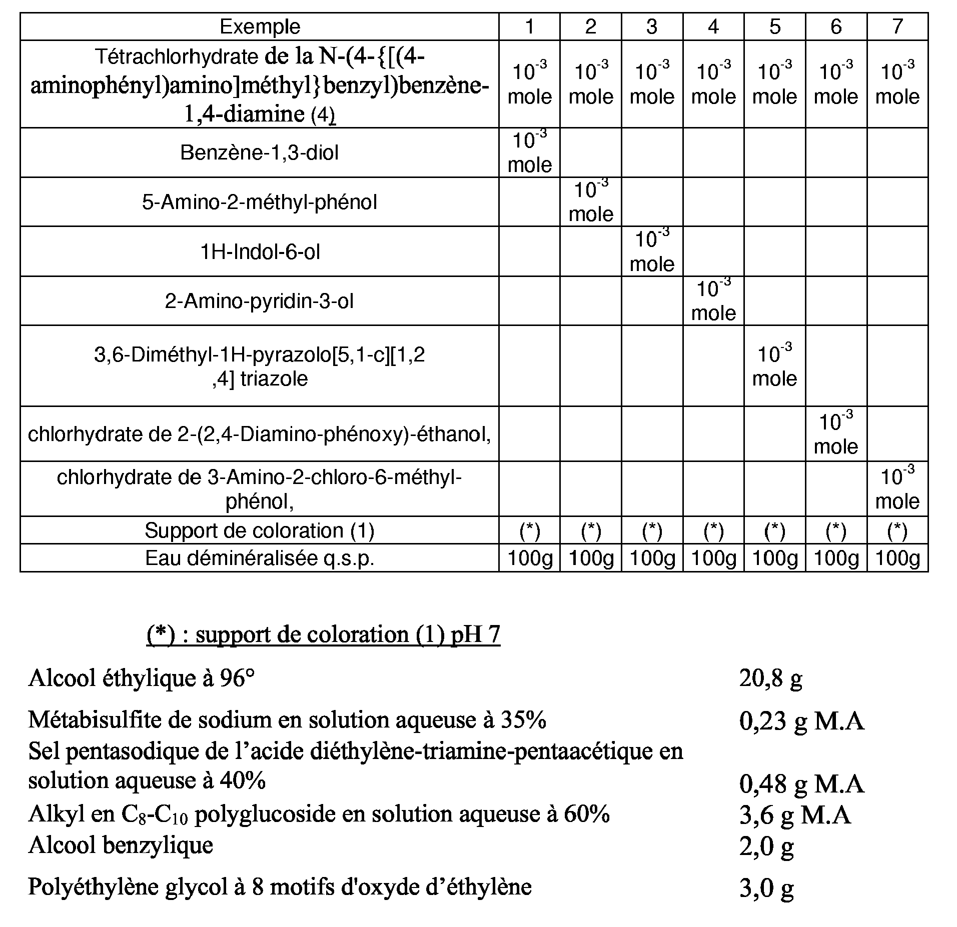

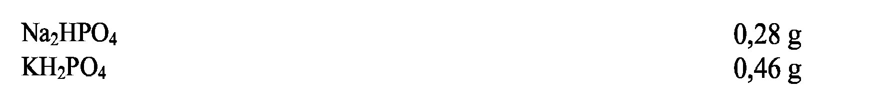

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.Les nuances obtenues figurent dans le tableau ci-dessous :

| Exemple | 1 | 2 | 3 | 4 | 5 | 6 | 7 |
|---|---|---|---|---|---|---|---|
| Nuance observée | jaune | rouge intense | brun rouge intense | brun rouge intense | orange | bleu intense | gris violet-rouge intense |

### Exemples 8 à 14 : coloration en milieu basique

Les compositions tinctoriales suivantes sont préparées :

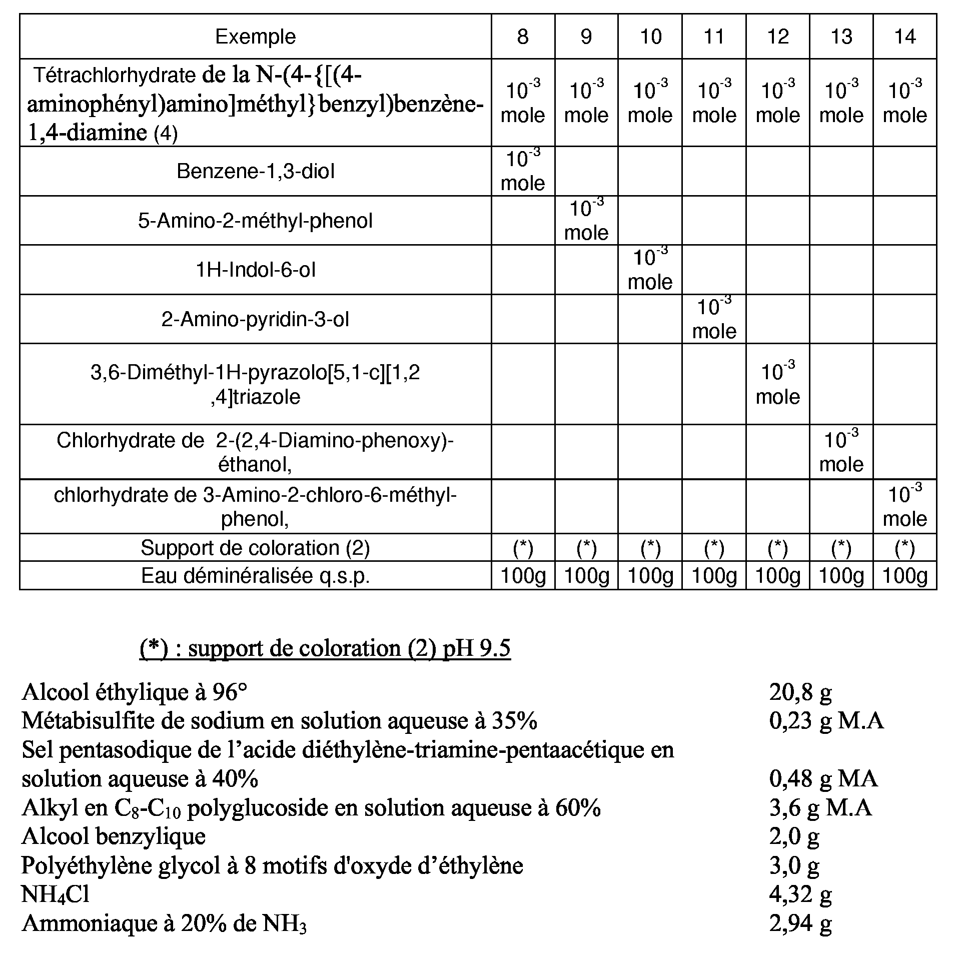

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 8 | 9 | 10 | 11 | 12 | 13 | 14 |
|---|---|---|---|---|---|---|---|
| Nuance observée | jaune | brun rouge intense | brun rouge intense | brun rouge intense | orangé | gris bleu intense | gris violet-rouge intense |

## Revendications

1. Composition cosmétique de coloration des fibres kératiniques comprenant, dans un milieu cosmétique approprié, au moins une base d'oxydation para-phénylènediamine de formule (I) suivante et ses sels d'addition correspondants : 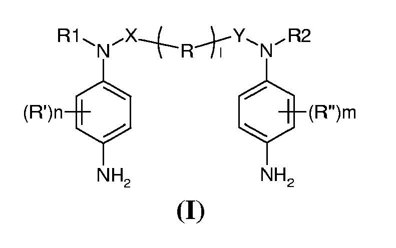 dans laquelle :
• R représente un cycle aromatique à 5 ou 6 chaînons, contenant éventuellement un ou plusieurs atomes d'azote, de soufre et d'oxygène ; ce cycle pouvant être substitué,
• R₁ et R₂ représentent, indépendamment l'un de l'autre,
- un atome d'hydrogène,
- un radical alkyle en C₁-C₆linéaire ou ramifié, pouvant être substitué par un ou plusieurs radicaux choisis parmi les radicaux hydroxy, alkoxy en C₁₋C₄, amino, mono-alkylamino en C₁-C₄, dialkylamino en C₁₋C_{4,}
• X et Y représentent, indépendamment l'un de l'autre, un radical alkylène en C₁-C₁₀ linéaire ou ramifié,
• R' et R" représentent, indépendamment l'un de l'autre,
- un radical alkyle en C₁-C₆,
- un radical alcoxy en C₁-C₆,
- un radical hydroxy-alcoxy en C₁-C₆,
- un radical alcoxy(C₁-C₆)alkyle en C₁-C₆,
- un radical mono ou poly-hydroxy alkyle en C₁-C₆,
• n et m représentent, indépendamment l'un de l'autre, un nombre entier variant de 0 à 4,
• 1 représente un nombre entier égal à 1 ou 2.

2. Composition selon la revendication 1 dans laquelle les para-phénylènediamines de formule (I) sont telles que R est un radical phénylène pouvant être substitué.

3. Composition selon la revendication 1 ou 2 dans laquelle les para-phénylènediamines de formule (I) sont telles R₁ et R₂ représentent indépendamment un atome d'hydrogène ou un groupe alkyle en C₁-C₄ pouvant être substitué.

4. Composition selon l'une quelconque des revendications précédentes dans laquelle X et Y représentent indépendamment un radical alkylène en C₁-C₃.

5. Composition selon l'une quelconque des revendications 1 à 4 dans laquelle le cycle R peut être substitué par un ou plusieurs radicaux choisis parmi les radicaux alkyles en C₁-C₄ ou hydroxyles ou hydroxyalkyles.

6. Composition selon l'une quelconque des revendications 1 à 5 dans laquelle les para-phénylènediamines de formule (I) sont choisies parmi :

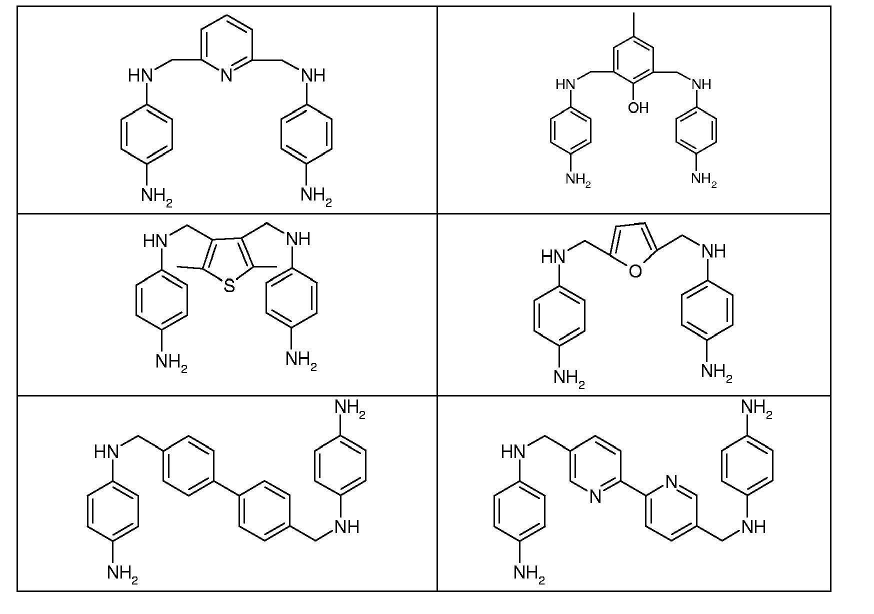

7. Composition selon l'une quelconque des revendications 1 à 6, **caractérisé en ce que** les sels d'addition avec un acide des para-phénylènediamines doubles de formule générale (I) sont choisis parmi l'acide chlorhydrique, l'acide bromhydrique, l'acide sulfurique, l'acide citrique, l'acide succinique, l'acide tartrique, l'acide lactates, l'acide para-toluènesulfonique, l'acide benzènesulfonique, l'acide phosphates et l'acide acétique, ces composés pouvant éventuellement être sous forme de solvates.

8. Composition de coloration selon l'une quelconque des revendications 1 à 7 dans laquelle la quantité en para-phénylènediamine de formule (I) est comprise entre 0,0001% et 20% en poids par rapport au poids total de la composition, de préférence entre 0,01% et 10%.

9. Composition selon l'une quelconque des revendications précédentes comprenant de plus au moins un coupleur d'oxydation.

10. Composition selon la revendication 9 dans laquelle les coupleurs d'oxydation sont choisis parmi les méta-phénylènediamines, les méta-aminophénols, les méta-diphénols, les coupleurs naphtaléniques et les coupleurs hétérocycliques ainsi que leurs sels d'addition.

11. Composition selon l'une quelconque des revendications 1 à 10 comprenant de plus au moins une base d'oxydation additionnelle différente des bases d'oxydation de formule (I).

12. Composition selon la revendication 10 dans laquelle les bases d'oxydation sont choisies parmi les para-phénylènediamines, les bis-phénylalkylènediamines, les para-aminophénols, les ortho-aminophénols et les bases hétérocycliques et leurs sels d'addition.

13. Composition selon l'une quelconque des revendications 1 à 12 comprenant au moins un colorant direct.

14. Composition selon l'une quelconque des revendications 1 à 13 dans laquelle le milieu approprié pour la coloration est constitué par de l'eau contenant éventuellement au moins un solvant organique.

15. Composition selon la revendication 14 dans laquelle le solvant organique est choisi parmi les alcools inférieurs en C₁-C₄, ramifiés ou non, ainsi que les alcools aromatiques et leurs mélanges.

16. Composition selon l'une quelconque des revendications 1 à 15 comprenant au moins un adjuvant cosmétique choisi parmi les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

17. Composition selon la revendication 16 **caractérisée en ce que** la quantité en adjuvant cosmétique est comprise pour chacun d'eux entre 0,01 et 20% en poids par rapport au poids total de la composition.

18. Composition selon l'une quelconque des revendications précédentes comprenant un agent oxydant.

19. Procédé de coloration des fibres kératiniques **caractérisée par le fait qu'**on applique sur les fibres au moins une composition selon l'une quelconque des revendications 1 à 18 pendant une durée suffisante pour développer la coloration désirée en présence d'un agent oxydant.

20. Dispositif à plusieurs compartiments dans lequel un premier compartiment contient une composition de coloration des fibres kératiniques telle que définie à l'une quelconque des revendications 1 à 18 et un deuxième compartiment contient un agent oxydant.

21. Para-phénylènediamine de formule (I) telle que définie selon l'une quelconque des revendications 1 à 7 à l'exception des composés

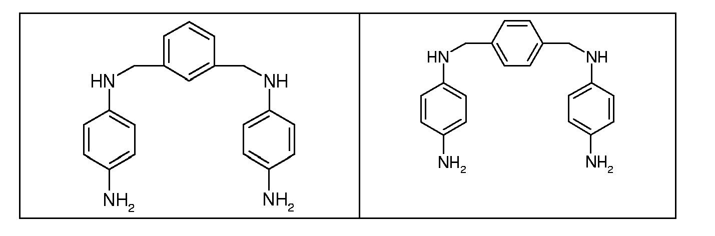

22. Composé nitré de formule (II) 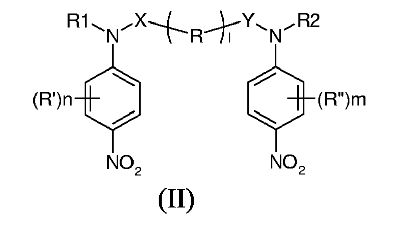 dans laquelle R₁, R₂, X, Y, R, R', R", n, m et 1 sont tels que définis aux revendications 1 à 7 à l'exception des composés

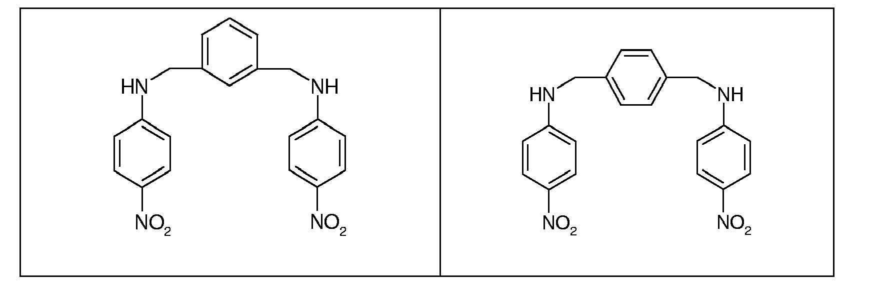

23. Procédé de préparation des para-phénylènediamines telles que définies à la revendication 21 par réduction des composés nitrés définis à la revendication 22.
